# EUROPEAN PATENT SPECIFICATION

(11) **EP 1 727 594 B1**
(45) Date of publication and mention of the grant of the patent: **20.07.2011**
(21) Application number: 05702009.1
(22) Date of filing: 24.01.2005
(51) Int. Cl.: A61N 1/39, A61N 1/372, A61N 1/375

(54) **A DEFIBRILLATOR DEVICE WITH AN ACTIVATING DEVICE AT ITS HANDLE**
DEFIBRILLATOR MIT AKTIVIERUNGSVORRICHTUNG AN DEM GRIFF
DISPOSITIF DEFIBRILLATEUR AYANT UN ELEMENT D'ACTIVATION SUR LA POIGNÉE

(30) Priority: 23.01.2004 GB 0401455; 28.01.2004 US 539335 P
(43) Date of publication of application: 06.12.2006
(73) Proprietor: Ixa Medical Products LLP, Cheltenham Gloucestershire GL50 1SS (GB)
(72) Inventor: HERBERT, Kevin J., c/o IXA Medical Products L.L.P., Cheltenham, Gloucestershire GL50 1SS (GB); MILLS, D. B., c/o IXA Medical Products L.L.P., Gloucestershire GL50 1SS (GB)
(74) Representative: Newell, William Joseph
(86) International application number: PCT/GB2005/000248
(87) International publication number: WO 2005/070497

(56) References cited:
- US-A1- 2001 031 927
- US-A1- 2003 088 291
- US-A1- 2003 208 237
- US-B1- 6 618 622

## Description

This invention relates to defibrillators, and in particular, but not exclusively, automatic or semi-automatic external defibrillators (AEDs). Further this invention relates to a defibrillator that is automatically activated or inactivated by, for example, touching or holding a sensory section or sections of the casing.

### BACKGROUND

In England only two to three people in every hundred survive Sudden Cardiac Death (SCD) compared with eight to nine in Scotland and eleven in the United States. In the United States, on average, 1000 people per day die; this translates into one death every two minutes.

Over 80% of SCD cases are caused by ventricular fibrillation ("VF"), in which the heart's muscle fibres contract without any coordination, resulting in a significantly reduced blood flow to the body. Currently, the most effective and widely used treatment for VF is electrical defibrillation, which applies an electrical shock through the patient's heart, clearing the abnormal electrical activity (a process called "defibrillation") by depolarising a critical mass of myocardial cells to allow spontaneous organized myocardial depolarisation to resume.

To be most effective, the defibrillation shock must be delivered to the patient within minutes of the onset of VF. It is well documented that defibrillation shocks delivered within one minute after the onset of VF achieve up to a 100% survival rate. However, statistically the survival rate falls by approximately 10% per minute and beyond 10 minutes, the survival rate approaches zero. Importantly, the more time that passes, the longer the brain is deprived of oxygen and the more likely that brain damage will result.

In the course of a rescue, even if the patient is discovered immediately, it can take a few minutes to retrieve the AED and a few additional minutes for the AED to diagnose and treat the patient. As can be appreciated, the less time required for an operator to activate and set up the AED, the better the chances that the patient will survive.

Government policies and growing awareness of AEDs has led to a greater proliferation of easily accessed public devices. This in turn has brought the devices to a wider public audience where an operator has a greater likelihood of having little of no training in the use of an AED. Even where the operator has had training he or she may well be unfamiliar with a particular brand of AED device and this can lead to confusion or even panic at the onset of a rescue trying to determine how to activate the AED.

Traditionally, it is considered universal that the on/off switch may use a "1" to indicate "on", and a "0" to indicate "off." However, the recognition of the on/off switch is often confusing to someone without prior knowledge or experience, particularly when under extreme stress. Furthermore, AEDs generally have had several buttons for performing differing functions such as SHOCK and ANALYSE and the location of an on/off switch can be confusing to find.

An AED is needed, that is capable of walking the operator through the stages of a rescue with the operator performing a minimum of functions in a very simple way and requiring no special knowledge of how to operate the device.

US Patent No. 6556864 describes a defibrillator that is automatically activated or inactivated by, for example, inserting or removing an object, such as a plug or a pin, from a receptacle. It, is stated that the object could be related to an electrode, whereby removing the electrode causes the device to turn on. However this system still requires an operator to know that in order to use the device he has to first remove an object such as the electrode. There is a risk that in operation the operator may waste valuable time looking for an obvious "ON" button as he may be familiar with devices having an ON button. This is especially true if the operator is panicking and is not aware of or does not pay attention to any displays instructing him to remove an object first.

US Patent No. 5645571 describes a device in which opening the lid covering the electrode compartment to expose the electrodes causes the AED to be powered ON; Again, this device requires the operator to have the knowledge that he has to first lift a lid to activate the device. Without prior knowledge valuable time could again be wasted.

US Published Application No. 20030120311 describes a device which automatically turns on when an operator removes it from a storage location. In this arrangement an AED is automatically activated without the operator requiring prior knowledge of performing some special act of removing or lifting something connected with the device and so overcomes the limitations of the arrangements described in the above two US patents. However, this arrangement requires that the AED remain in a storage case at all times when not in use and hence adds to the weight and bulkiness of the device when in when portability is a key issue, for example where trained operators at a concert event or show carry the device across the shoulders in a carry bag.

US2003/208237 discloses, according to the preamble of claim 1, an AED which has a housing with a lid which when closed covers at least a portion of the user interface. A dual function on/off button is configured so that when an operator depresses the button, it causes a switch to close to activate the device and further unlatches the lid to open it.

US2003/088291, US2001/0B1927 and US-B1-6618622 each disclose medical devices which include touch screens or similar devices.

### SUMMARY OF THE INVENTION

Accordingly, we have designed an improved defibrillator device which changes its operational state when touched or handled on a sensory section or sections of the casing and particularly without requiring the operator to determine the location of an ON/OFF switch to activate it. Thus in particular embodiments of this invention, the defibrillator changes its operational state when touched or handled on a sensory section or sections of the casing.

In one aspect, this invention provides a defibrillator device comprising a casing including a handle region and further including a handle region containing electrical circuitry for generating in use a defibrillation voltage for application to a patient, a control system for controlling operation of said defibrillator device, and a detector means which is associated with at least one region of said casing and which is responsive to at least one of touching or handling by, or proximity of, an operator, said system being responsive in use to said detector means to change the operational status of said defibrillator device from a first state to a second state on detection of an operator, wherein said detector means Is associated with said handle or an adjacent region on said casing whereby the operational status of the device is changed in use when an operator grabs the handle.

In this way the operational status of the defibrillator device changes without requiring the operator to identify the whereabouts of the ON/OFF switch and also without requiring any specific action such as the removal of items, opening of a casing or removal from a casing, etc. Thus in preferred embodiments the change in operational status is effected without requiring any specific actions normally associated with turning on the defibrillator.

We have found that, in practice, where the defibrillator is provided with a handle, the majority of operators will grasp the handle on reaching the defibrillator device. Where the detector means is associated with the handle, it may take many forms but it is preferred that It is arranged elative to the handle such that it detects or trips when the operator grips the handle. Here the detector means may utilise a switch but activation thereof does not require the operator to identify the switch as such. Alternatively, other types of detector means may be used such as membrane switches, capacitive switches, IR detectors etc.

Generally, the detector means may comprise a plurality of detector devices located at selected space locations on the casing to provide touch-sensitive or proximity-sensitive sensory regions.

The detector means may comprise any suitable electrical, mechanical, electro-magnetic, thermal (e.g. IR), capacitive or other sensors capable of detecting the presence of a operator either passively or actively. Thus a typical detector may comprise a movable element associated with, e.g., the handle, with movement thereof being detected by a micro-switch, or it may comprise a capacitive switch.

Accordingly in some arrangements the sensor may detect physical contact by an operator whereas in others the sensor may detect the operator being within a pre-set proximity.

Preferably, sustained detection of an operator beyond the pre-set period causes the control system to change the operational status of the defibrillator device to a different operational state which may be the same or different to the first operational state.

The first operational state may be an OFF mode; alternatively it may be a sleep mode. Such a mode is designed to conserve power by deactivating all non-essential components until absolutely needed. In use, this mode may be controlled by a counter and interrupt circuit. When entering the sleep mode all non-essential and non-volatile components are turned off and the counter is activated. Thus at a predetermined time the counter turns on and triggers the interrupt; essential components required to maintain the operating reliability of the device are activated (i.e. the unit "wakes up") to perform a self check. The interrupt may also be triggered by an external factor such as a switch or other form of detector (heat, movement etc.). Such sleep modes are well known in the art and can maintain the unit in a state of readiness, but at less than 0.5% of the operating power.

The second state may be an ON state or, where the control system is operable to effect a self-test routine, the second state may be a self-test organisational state.

The control system may be responsive to the absence of touching, handling or proximity of an operator within a pre-set period to change the operational status of the device to a further state which may be the same or different to the first state. In this arrangement the defibrillator may be returned to an OFF or sleep mode after a pre-determined absence, or the device may change from an ON or self-test operational state to a further operational state prior to delivery of the defibrillation voltage.

Preferably, the defibrillator device includes means for issuing instructions to an operator following detection by said detector means; the instructions may be verbal, issued via a loudspeaker or the like, and/or visual issued by a display on the defibrillator device.

Preferably the control means is operable to issue wan instruction to the operator to connect a further item of equipment, such as a pair of electrodes, on detecting absence of an operator following the change from said first operational state to second operational state.

Additionally, or alternatively, the control system may issue an instruction for the operator to touch or trip a further sensor on detecting absence of the operator following said change from said first operational state to said second operational state.

The defibrillator device may include one or more attitude sensors for detecting the attitude of the casing and for supplying the corresponding attitude signals to said control system. This allows the control system to determine the orientation of the defibrillator device, with a certain orientation being associated with the defibrillator device being carried, and another orientation being associated with it being laid on the ground right way up, and so on.

In one arrangement, after application of a defibrillation voltage in use to a patient, detection by said detector means of the presence or absence of operator (or a pre-determined sequence of presence/absence) causes the device to provide and/or store post-rescue data.

In another arrangement, where the defibrillator device includes means for storing data relating to the operation of the device following the application of a defibrillation voltage to a patient, said control system may be responsive to a signal from said detector means to apply a compression algorithm to said stored data.

Accordingly the illustrated embodiment provides an improved automated external defibrillator (AED), which changes its operational state when touched or handled on a sensory section or sections of the casing. As illustrated this section comprises a handle area in the casing. However the section could be positioned anywhere about the device or alternatively multiple sensory sections could be located about the AED casing or the entire AED casing itself could form the sensory section.

The sensory section may consist of an electrical, mechanical, electromagnetic, thermal or otherwise sensor capable of detecting the presence of an object, such as an operator's hand either passively or actively. As illustrated the object must be touching the sensor section in order to be detected, but in alternative embodiments the object could be within a certain proximity of the casing in order to be detected.

Upon detection the defibrillator changes its operational state appropriate for the rescue. In the preferred embodiment the defibrillator changes from a sleep mode to an active mode. In this embodiment this is followed by audio and visual commands instructing the operator to what further actions he should do in order to carry out a rescue.

In other embodiments the defibrillator might turn from an off state to an on state. In another embodiment, detection results in the defibrillator's operational mode entering a self-test state whereby the defibrillator checks that it is performing correctly. It may then enter an on state automatically or as a result of a continued detection. Thus it become possible to check the defibrillator status without entering an on state by the operator touching the sensor section of the device and letting go.

### DESCRIPTION OF THE PREFERRED EMBODIMENT

Whilst the invention has been described above it extends to any inventive combination of the features set out above or in the following description.

The invention may be performed in various ways, and, by way of example only, an embodiment thereof will now be described by way of example only, reference being made to the accompanying drawings in which:-
Figure 1 is a schematic view of an embodiment of a defibrillator in accordance with this invention, and
Figure 2 is a block diagram illustrating the control system of the defibrillator of Figure 1.

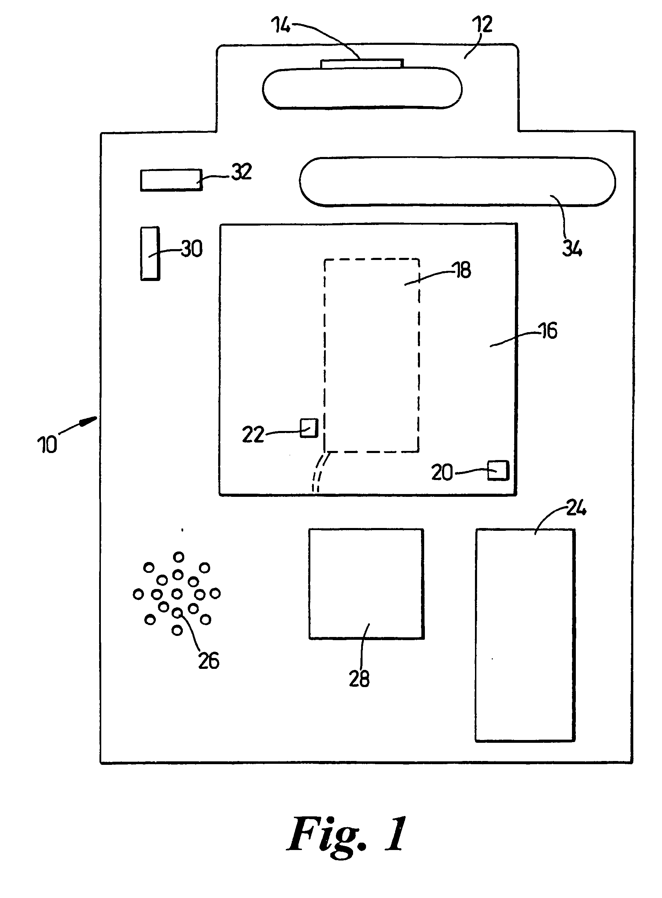

Referring initially to Figure 1, a semi-automatic automated external defibrillator in accordance with the invention is illustrated. The defibrillator includes a plastic case 10 with a carrying handle 12 on the top portion. The plastic casing 10 contains the storage battery control circuitry and associated equipment for the defibrillator. Associated with the handle 12 of the defibrillator is a handle switch 14 which, in this embodiment, is a capacitive switch although it could be a micro-switch. The casing 10 includes a compartment 16 for the storage of electrode pads 18 and appropriate sensors 20 and 22, may be provided to detect opening of the electrode compartment 16 and/or removal of the electrodes. The defibrillator includes a visual display screen 24 and a loudspeaker 26 from which audio or visual instructions may be issued. The casing also includes an 'activation' button 28 and, internally of the casing, respective attitude sensors 30 and 32 for detecting the attitude of the casing relative to orthogonal axes. Also associated with casing is a further touch-sensitive region 34.

In operation, the defibrillator is arranged such that it switches from an OFF state to an ON state as soon as an operator grabs the handle.

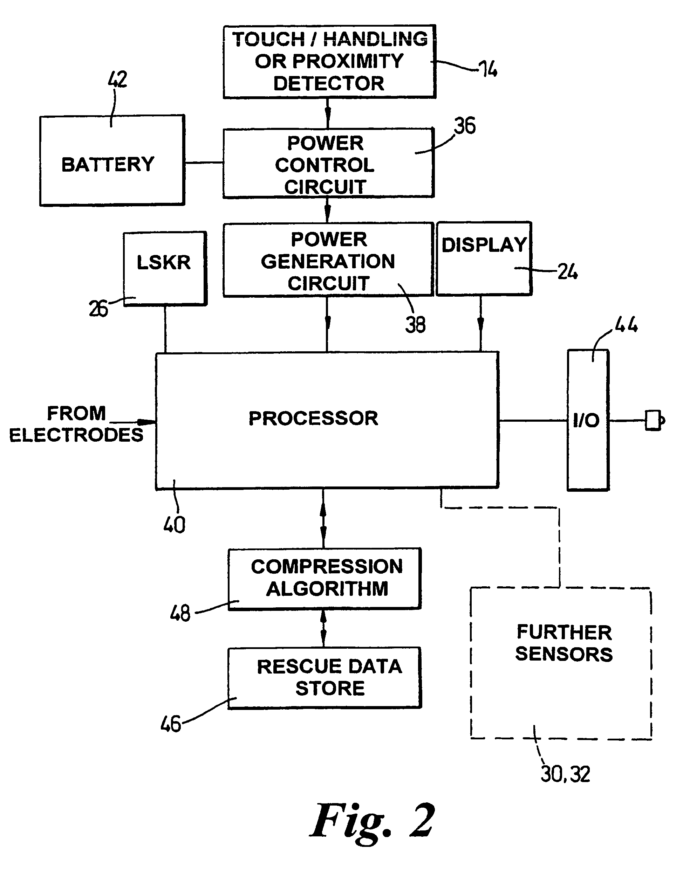

One example of a control system for the defibrillator of Figure 1 is shown in Figure 2.

The rescue mode operation of the defibrillator is initiated when an operator grabs the handle 14 on the top portion in order to carry or move the defibrillator 10. The switch 14 mounted within the handle casing detects the grabbing of the handle and effectively functions as an on/off switch. In response to this action, a power control circuit 36 activates a power generation circuit 388 and initiates a rescue mode operation of a processor 40. The processor 40 then begins a rescue mode operation by performing a self-test and then initiates the generation of an audible voice prompt via the loudspeaker 26 "Take to rescue site and remove electrode pads." Such audio prompting may be reinforced or replaced by visual elements e.g. on a display 24 without departing from the scope of the patent.

Once the processor 40 has performed the self-test and on the condition that the defibrillator is functioning correctly, the processor then repeats the audio prompt, "Take to rescue site and remove pads."

Assuming that the handle is still being used to carry the defibrillator, the audio prompting will continue to repeal at a predetermined rate unless removal of the electrodes 18 from the defibrillator casing 10 has been detected by sensor 22, or removal of the electrodes upon the patient. If desired, at a defined time following the initial grasping of the handle 12, the audio prompting may become less frequent or cease altogether. This would prevent the battery powering the defibrillator system from being unnecessarily depleted and will also prevent the device becoming a audible nuisance if it were just being transported without any form of carry case or bag. Should the device be placed down and then picked up again, thereby reactivating the handle, the process would start from the beginning.

Upon removing the electrodes 18 from the casing and/or upon removing the electrodes from their associated packaging, the processor 40 guides the operator through a sequence of actions required to enable the AED to achieve a stage capable of performing an ECG analysis and subsequent rescue shock if necessary. This typically involves placing the electrodes 18 upon the patients' chest, checking the airway, breathing and circulation of the patient and monitoring the impedance between the electrodes for future reference if a shock were to be required.

In another embodiment, if the processor detects that the defibrillator has been activated and then deactivated, e.g. by the operator grabbing the handle and subsequently placing the unit down and releasing the handle, it may issue verbal instructions to guide the operator to attach a pair of defibrillator electrodes 18 not presently connected. Furthermore in another variation, the processor may detect when the electrode pads are connected and when they are not automatically, and issue a suitable announcement such as e.g. "unlit in travel mode" or "unit OK" as appropriate. In a third embodiment the defibrillator may guide the operator to touch a further "activation" or "continue rescue" button 28 in order for the rescue to proceed.

In a fourth embodiment the defibrillator may have further sensors to detect at which stage the rescue is currently at and determine its audible or visual prompts accordingly. Such sensors may be attitude sensors 30, 32 capable of detecting whether the device is vertical or horizontal, and whether the device has been mistakenly placed on the ground the wrong way round. These sensors may only activate when the handle activation button 14 is released, or alternatively may be active for a predetermined time relative to the activation of the handle button 14.

Naturally after the completion of a rescue the operator will activate the handle sensor 14 again when attempting to place the defibrillator back in storage or transport the unit with the patient to a nearby hospital facility.

This post-rescue activation will cause the processor 40 to collect and to provide post-rescue data. This way includes instructions as to how to download or transfer any rescue data that may be held in memory, or as to the state of the battery and whether a recharge or replace will be necessary, or instruct the operator to replace the electrodes 18 used during the rescue.

Furthermore, the defibrillator contains an output interface 44 to enable any stored data to be transferred to a remote or local data storage or processing facility either directly by electrical cable, infra-red communication or wireless applications or via connection to a modem and subsequently to a communication network (for example across an internet or intranet connection). Such data may include collected data during a rescue such as ECG, impedance, respiration, defibrillation data and audio or visual data collected by a microphone or camera as well as data collected during the defibrillator 's lifetime (self test data, event history etc).

Traditionally such data will require a large amount of memory and transfer across a data connection can take anywhere up to an hour if the connection is not high speed or if the data connection has a large amount of data traffic at that time. As can be appreciated requiring such a large amount of time to study any urgent data is not acceptable.

To reduce this data transfer time post-rescue, gripping the handle 12 after use signals the defibrillator to compress the data stored in a memory 46 using a compression algorithm 48 prior to the transfer of data. Such a compression operation on such a large amount of data can take up to ten minutes or so. Hence such an essential operation is performed while the defibrillator is in transit to either a carry bag or being taken with the patient to a hospital and the unit is confirmed to not be in a rescue state (i.e the unit has been picked up). Should the handle switch 14 again be activated and new electrodes used to perform a second rescue, the compression can be halted and data relating to a new rescue stored in the available memory.

Following the release of the post-rescue handle activation the defibrillator 10 may perform an additional self test to ensure that no damage occurred during transit.

In summary therefore the described embodiment and the various possible modifications illustrate an example of an automated or semi-automated defibrillator that automatically changes the operation mode, prior to any intended use, when an operator contacts a section or sections of the casing. In one embodiment operation mode of the defibrillator changes from a sleep mode to an on mode; in another, the operation mode of the defibrillator may change from an off mode to an on mode.

Furthermore, the defibrillator can be designed to automatically change the operational mode when the operator has no contact with a section or sections of the casing for a predetermined amount of time. Thus the operational mode of the defibrillator may change from an on mode to a sleep mode or alternatively from an on mode to an off mode.

This automatic activation has been found to often decrease the time it takes the operator-particularly an inexperienced or anxious operator-to set up and use the AED to resuscitate a patient in cardiac arrest. Furthermore, the operational mode of the defibrillator can be configured to change to a self-test mode when an operator contacts a section or sections of the casing prior to or following any intended use of the defibrillator. Thus the tested functions could include the presence and interconnection of defibrillator electrodes, battery charge state and the operability of the high voltage circuit. Visual and audible indicators are used to alert an operator if faults are identified. A record of each self-test may be stored in memory, and can be subsequently retrieved through a communications port or transmitted to a communications device via a transmitter module.

## Claims

1. A defibrillator device comprising a casing (10) having a carrying handle (12) and further containing electrical circuitry (36, 38) for generating in use a defibrillation voltage for application to a patient, a control system (40) for controlling operation of said defibrillator device, and a detector means (14) which is associated with at least one region (12) of said casing and which is responsive to at least one of touching by, handling by, or proximity of, an operator, said control system (40) being responsive in use to said detector means (14) to change the operational status of said defibrillator device from a first state to a second state on detection of an operator, **characterised in that** said detector means (14) is associated with said carrying handle (12) or an adjacent region on said casing, whereby the operational status of the device is changed in use when the handle is grabbed by an operator.

2. A defibrillator device according to Claim 1, wherein said detector means (14) is one or more detector elements selected from the group comprising microswitches, IR detectors, capacitive sensors, membrane switches.

3. A defibrillator device according to Claim 1 or Claim 2, wherein said detector means (14) defines a sensing region on said casing.

4. A defibrillator device according to any of the preceding Claims, wherein said detector means (14) comprises a proximity detector.

5. A defibrillator device according to Claims 1 to 3, wherein said detector means (14) comprises a contact detector.

6. A defibrillator device according to any of the preceding Claims, wherein said control system (40) is operable on sustained detection of an operator to change the operational state of said defibrillator device from said second state to a different state.

7. A defibrillator device according to any of the preceding Claims, wherein said first state is an Off state.

8. A defibrillator device according to any of Claims 1 to 6, wherein said first state is a quiescent or sleep state.

9. A defibrillator device according to Claim 8, wherein said control system (40) is operational periodically to initiate a self-check routine during said quiescent or sleep state.

10. A defibrillator device according to any of the preceding Claims, wherein said second state is an On state.

11. A defibrillator device according to any of Claims 1 to 9, wherein said second state is a self-test state in which said control system initiates a self test routine.

12. A defibrillator device according to any of the preceding Claims, wherein following detection of an operator and changing of the operational status of the defibrillator to said second state, the control system (40) is operable to change the operational status of the defibrillator device to a different state on sustained detection of said operator beyond a preset period.

13. A defibrillator device according to any of the preceding Claims, wherein, following detection of an operator, the control system (40) is operable to change the operational status of the device to a different state if no further detection is detected within a preset period.

14. A defibrillator device according to any of the preceding Claims, wherein the defibrillator device includes means (40, 24, 26) for issuing instructions to an operator following detection by said detector means.

15. A defibrillator device according to Claim 14, wherein said means for issuing instructions comprises at least one of a loudspeaker (26) or a display (24) on the defibrillator device.

16. A defibrillator device according to Claim 15, wherein the control system (40) is operable to issue an instruction to the operator to connect a further item of equipment, on detecting absence of an operator following the change from said first operational state to a second operational state.

17. A defibrillator device according to Claims 15 or Claim 16, wherein the control system (40) is operable to issue an instruction for the operator to touch or trip a further sensor (28) on detecting absence of the operator following said change from said first operational state to said second operational state.

18. A defibrillator device according to any of the preceding Claims, which includes one or more attitude sensors (30, 32) for detecting the attitude of the casing and for supplying the corresponding attitude signals to said control system.

19. A defibrillator device according to any of the preceding Claims, wherein, after application of a defibrillation voltage in use to a patient, detection by said detector means (14) of the presence or absence of operator causes the device to provide and/or store post-rescue data.

20. A defibrillator device according to any of the preceding Claims, wherein the defibrillator device includes means (48) for storing data relating to the operation of the device following the application of a defibrillation voltage to a patient, and said control system is responsive to a signal from said detector means (14) to apply a compression algorithm to said stored data.

## Patentansprüche

1. Defibrillator, umfassend ein Gehäuse (10) mit einem Traggriff (12) und enthaltend eine elektrische Schaltung (36, 38) zur Erzeugung bei Benutzung eine Defibrillator-Spannung zur Anwendung an einem Patienten, ferner umfassend ein Steuersystem (40) zur Steuerung des Betriebs des Defibrillators, und einen Detektor (14), der wenigstens einem Bereich (12) des Gehäuses zugeordnet ist und wenigstens auf Berühren oder Handhaben oder Nähern einer Bedienungsperson anspricht, wobei das Steuersystem (14) im Betriebszustand auf den Detektor (14) reagiert, um den Betriebszustand des Defibrillators von einem ersten Nachweiszustand zu einem zweiten Nachweiszustand durch eine Betriebsperson zu ändern, **dadurch gekennzeichnet, daß** der Detektor (14) mit dem Traggriff (12) oder einem benachbarten Bereich an dem Gehäuse verbunden ist, wodurch der Betriebszustand des Defibrillators bei Benutzung sich ändert, sobald der Traggriff von einer Bedienungsperson ergriffen wird.

2. Defibrillator nach Anspruch 1, **dadurch gekennzeichnet, daß** der Detektor (14) ein oder mehrere Detektorelemente aufweist, die aus der Gruppe ausgewählt sind, welche Mikroschalter, Infrarot-Detektoren, kapazitive Sensoren und Membranschalter beinhaltet.

3. Defibrillator nach Anspruch 1 oder 2, **dadurch gekennzeichnet, daß** der Detektor (14) auf dem Gehäuse einen Sensorbereich aufweist.

4. Defibrillator nach einem der vorhergehenden Ansprüche, **dadurch gekennzeichnet, daß** der Detektor (14) ein Näherungsdetektor ist.

5. Defibrillator nach den Ansprüchen 1 bis 3, **dadurch gekennzeichnet, daß** der Detektor (14) einen Berührungsdetektor bildet.

6. Defibrillator nach einem der vorhergehenden Ansprüche, **dadurch gekennzeichnet, daß** das Steuersystem (14) mit unterstützender Ermittlung einer Bedienungsperson arbeitet, um den Betriebszustand des Defibrillators von dem zweiten Zustand in einen anderen Zustand zu ändern.

7. Defibrillator nach einem der vorhergehenden Ansprüche, **dadurch gekennzeichnet, daß** der erste Zustand ein Aus-Zustand ist.

8. Defibrillator nach einem der Ansprüche 1 bis 6, **dadurch gekennzeichnet, daß** der erste Zustand ein Ruhe- oder Schlafzustand ist.

9. Defibrillator nach Anspruch 8, **dadurch gekennzeichnet, daß** das Steuersystem (40) periodisch arbeitet, um während des Ruhe- oder Schlafzustandes einen Selbstkontrollgang zu veranlassen.

10. Defibrillator nach einem der vorhergehenden Ansprüche, **dadurch gekennzeichnet, daß** der zweite Zustand ein EIN-Zustand ist.

11. Defibrillator nach einem der Ansprüche 1 bis 9, **dadurch gekennzeichnet, daß** der zweite Zustand ein Selbstüberprüfungs-Zustand ist, in dem das Steuersystem einen Selbstüberprüfungsgang veranlaßt.

12. Defibrillator nach einem der vorhergehenden Ansprüche, **dadurch gekennzeichnet, daß** auf die Feststellung einer Bedienungsperson und den Wechsel des Betriebszustandes des Defibrillators in den zweiten Zustand das Steuersystem (40) dahingehend arbeitet, daß der Betriebszustand des Defibrillators mit unterstützender Ermittlung der Bedienungsperson über eine eingestellte Zeitspanne hinaus in einen anderen Zustand geändert wird.

13. Defibrillator nach einem der vorhergehenden Ansprüche, **dadurch gekennzeichnet, daß** nach Feststellung einer Bedienungsperson das Steuersystem (40) so arbeitet, daß der Betriebszustand des Defibrillators in einen anderen Zustand geändert wird, wenn in einer vorgegebenen Zeitspanne keine weitere Ermittlung erfolgt.

14. Defibrillator nach einem der vorhergehenden Ansprüche, **dadurch gekennzeichnet, daß** der Defibrillator Einrichtungen (40, 24,, 26) aufweist, mit denen auf Feststellung des Detektors hin Instruktionen an eine Bedienungsperson abgegeben werden.

15. Defibrillator nach Anspruch 14, **dadurch gekennzeichnet, daß** die Einrichtungen zur Abgabe von Instruktionen wenigstens einen Lautsprecher (26) und ein Display (24) auf dem Defibrillator aufweisen.

16. Defibrillator nach Anspruch 15, **dadurch gekennzeichnet, daß** das Steuersystem (40) so betreibbar ist, daß es eine Instruktion an die Bedienungsperson aussendet, um ein weiteres Teil einer Ausrüstung anzuschließen, wenn nach dem Wechsel von dem ersten Betriebszustand in einen zweiten Betriebszustand die Abwesenheit einer Bedienungsperson festgestellt wird.

17. Defibrillator nach Anspruch 15 oder 16, **dadurch gekennzeichnet, daß** das Steuersystem (40) dahingehend arbeitet, daß es für die Bedienungsperson eine Instruktion ausgibt, einen weiteren Sensor (28) zu berühren oder zu schalten, wenn die Abwesenheit der Bedienungsperson festgestellt wird, und zwar nach dem Wechsel von dem ersten Betriebszustand zu dem zweiten Betriebszustand.

18. Defibrillator nach einem der vorhergehenden Ansprüche, **gekennzeichnet durch** einen oder mehrere Stellungssensoren (30, 32) zur Ermittlung der Stellung des Gehäuses und zur Abgabe der entsprechenden Stellungssignale an das Steuersystem.

19. Defibrillator nach einem der vorhergehenden Ansprüche, **dadurch gekennzeichnet, daß** nach Anlegen einer Spannung eines in Benutzung befindlichen Defibrillators an einen Patienten die von dem Detektor (14) gelieferte Feststellung des Vorhandenseins oder Nichtvorhandenseins der Bedienungsperson den Defibrillator veranlaßt, nachträgliche Rettungsdaten zu liefern und / oder zu speichern.

20. Defibrillator nach einem der vorhergehenden Ansprüche, **dadurch gekennzeichnet, daß** der Defibrillator eine Einrichtung (46) zum Speichern von Daten aufweist, die sich auf den Betrieb des Defibrillators beziehen, der auf die Anwendung einer Defibrillator-Spannung auf einen Patienten folgt, und daß das Steuersystem auf ein von dem Detektor (14) kommendes Signal reagiert, um an die gespeicherten Daten einen Verdichtungsalgorithmus zu liefern.

## Revendications

1. Dispositif défibrillateur comprenant une enveloppe (10) ayant une poignée de transport (12) et contenant en outre un ensemble de circuits électriques (36, 38) destiné à générer, en utilisation, une tension de défibrillation pour une application à un patient, un système de commande (40) destiné à commander le fonctionnement dudit dispositif défibrillateur, et un moyen de détection (14) qui est associé à au moins une zone (12) de ladite enveloppe et qui est réactif à au moins l'un parmi le toucher, la manipulation ou la proximité d'un opérateur, ledit système de commande (40) étant réactif, en utilisation, audit moyen de détection (14) pour changer l'état de fonctionnement dudit dispositif défibrillateur d'un premier état à un second état lors de la détection d'un opérateur, **caractérisé par le fait que** ledit moyen de détection (14) est associé à ladite poignée de transport (12) ou à une zone adjacente sur ladite enveloppe, ce par quoi l'état de fonctionnement du dispositif est changé en utilisation lorsque la poignée est saisie par un opérateur.

2. Dispositif défibrillateur selon la revendication 1, dans lequel ledit moyen de détection (14) est un ou plusieurs éléments de détection choisis dans le groupe comprenant les micro-interrupteur, les détecteurs à infrarouge, les capteurs capacitif, les interrupteurs tactiles.

3. Dispositif défibrillateur selon l'une des revendications 1 ou 2, dans lequel ledit moyen de détection (14) définit une région de détection sur ladite enveloppe.

4. Dispositif défibrillateur selon l'une quelconque des revendications précédentes, dans lequel ledit moyen de détection (14) comprend un détecteur de proximité.

5. Dispositif défibrillateur selon l'une des revendications 1 à 3, dans lequel ledit moyen de détection (14) comprend un détecteur à contact.

6. Dispositif défibrillateur selon l'une quelconque des revendications précédentes, dans lequel ledit système de commande (40) est actionnable lors d'une détection prolongée d'un opérateur pour changer l'état de fonctionnement dudit dispositif défibrillateur dudit second état à un état différent.

7. Dispositif défibrillateur selon l'une quelconque des revendications précédentes, dans lequel ledit premier état est un état arrêt.

8. Dispositif défibrillateur selon l'une quelconque des revendications 1 à 6, dans lequel ledit premier état est un état de repos ou de veille.

9. Dispositif défibrillateur selon la revendication 8, dans lequel ledit système de commande (40) fonctionne périodiquement pour lancer une routine de vérification automatique pendant ledit état de repos ou de veille.

10. Dispositif défibrillateur selon l'une quelconque des revendications précédentes, dans lequel ledit second état est un état marche.

11. Dispositif défibrillateur selon l'une quelconque des revendications 1 à 9, dans lequel ledit second état est un état d'essai automatique dans lequel ledit système de commande lance une routine d'essai automatique.

12. Dispositif défibrillateur selon l'une quelconque des revendications précédentes, dans lequel, à la suite de la détection d'un opérateur et du changement de l'état de fonctionnement du défibrillateur audit second état, le système de commande (40) est actionnable pour changer l'état de fonctionnement du dispositif défibrillateur à un état différent lors de la détection prolongée dudit opérateur au-delà d'une période prédéfinie.

13. Dispositif défibrillateur selon l'une quelconque des revendications précédentes, dans lequel, à la suite de la détection d'un opérateur, le système de commande (40) est actionnable pour changer l'état de fonctionnement du dispositif à un état différent si aucune autre détection n'est détectée en l'espace d'une période prédéfinie.

14. Dispositif défibrillateur selon l'une quelconque des revendications précédentes, dans lequel le dispositif défibrillateur comprend des moyens (40, 24, 26) d'émission des instructions à un opérateur à la suite d'une détection par ledit moyen de détection.

15. Dispositif défibrillateur selon la revendication 14, dans lequel lesdits moyens d'émission d'instructions comprennent au moins l'un parmi un haut-parleur (26) ou un dispositif d'affichage (24) sur le dispositif défibrillateur.

16. Dispositif défibrillateur selon la revendication 15, dans lequel le système de commande (40) est actionnable pour émettre une instruction à l'opérateur de relier un autre article d'équipement, lors de la détection de l'absence d'un opérateur à la suite du changement dudit premier état de fonctionnement à un second état de fonctionnement.

17. Dispositif défibrillateur selon l'une des revendications 15 ou 16, dans lequel le système de commande (40) est actionnable pour émettre une instruction à l'opérateur de toucher ou déclencher un autre capteur (28) lors de la détection de l'absence de l'opérateur à la suite dudit changement dudit premier état de fonctionnement audit second état de fonctionnement.

18. Dispositif défibrillateur selon l'une quelconque des revendications précédentes, lequel comprend un ou plusieurs capteurs d'orientation (30, 32) destinés à détecter l'orientation de l'enveloppe et à fournir les signaux d'orientation correspondants audit système de commande.

19. Dispositif défibrillateur selon l'une quelconque des revendications précédentes, dans lequel, après l'application, en utilisation, d'une tension de défibrillation à un patient, la détection par ledit moyen de détection (14) de la présence ou de l'absence d'un opérateur amène le dispositif à délivrer et/ou à stocker des données post-secours.

20. Dispositif défibrillateur selon l'une quelconque des revendications précédentes, dans lequel le dispositif défibrillateur comprend des moyens (46) destinés à stocker des données relatives au fonctionnement du dispositif à la suite de l'application d'une tension de défibrillation à un patient, et ledit système de commande est réactif à un signal provenant dudit moyen de détection (14) pour appliquer un algorithme de compression auxdites données stockées.
